# EUROPEAN PATENT APPLICATION

(11) **EP 0 729 763 A2**
(43) Date of publication of application: **04.09.1996**
(21) Application number: 95120706.7
(22) Date of filing: 29.12.1995
(51) Int. Cl.: A61M 11/02

(54) **Aerosol therapy apparatus**

(30) Priority: 28.02.1995 IT MI950384
(71) Applicant: MIAT S.P.A., I-20159 Milan (IT); MED 2000 S.r.l, 25010 Pozzolengo (Brescia) (IT)
(72) Inventor: Fraccaroli, Nicola, I-25015 Desenzano del Garda, Brescia (IT)
(74) Representative: Giambrocono, Alfonso, Dr. Ing.

(57) **Abstract**

An aerosol therapy apparatus (10) comprises an atomization chamber (14) in which the dose of liquid medicament to be atomized is placed, in the chamber (14) there being provided both an air intake aperture (16) and a delivery aperture (18) through which the atomized medicament stream leaves. The medicament atomization device comprises a disc-shaped element (20) positioned within the chamber (14) in such a manner as to dip into the liquid medicament contained in it, and means (24) for rotating the disc element (20) about its axis (22) at a high r.p.m. The rotational speed of the disc element and its surface roughness are chosen such as to obtain the required atomization of the medicament.

## Description

This invention relates to an aerosol therapy apparatus, ie an apparatus for producing an atomized medicament stream to be inhaled by a patient.

Traditional aerosol therapy apparatus are known to consist basically of a small compressor the purpose of which is to generate an air stream, and a glass or plastic bulb through which said air stream is passed, the bulb having a special shape such that the air stream passing through it atomizes medicament liquid previously fed into it.

Such a type of aerosol therapy apparatus has certain drawbacks. A first drawback is due to the fact that the compressor provided with the apparatus comprises contacting metal parts subject to mutual rubbing. This results in the production of metal powder which, although of minimum quantity, always represents an undesirable substance which is inhaled by the patient and could contaminate the medicament. The compressor also represents a source of noise, which can be annoying. Finally, it is well known that such aerosol therapy apparatus have a rather low atomization rate, so that the delivery of a dose of medicament engages the patient for a considerable time (even in excess of a quarter of an hour).

To reduce the medicament delivery time, ultrasonic aerosol therapy apparatus have been provided. In these, the medicament is atomized by exposing the liquid mass to ultrasound produced by a suitable device. Such known apparatus are however rather delicate and require special technical expedients both with regard to the oscillator and with regard to the ultrasound transducer to prevent the piezoelectric ceramic element of the ultrasound generating device becoming consumed within a short time. Furthermore in such apparatus the atomization rate varies significantly with the density of the liquid medicament to be atomized, so that denser or oily medicaments require a substantially longer atomization time.

An object of the present invention is to overcome the aforesaid drawbacks of known aerosol therapy apparatus.

A further object of the invention is to provide an aerosol therapy apparatus which has an atomization rate substantially higher than that of known apparatus.

These objects are attained according to the present invention by an aerosol therapy apparatus comprising an atomization chamber in which the dose of liquid medicament to be atomized is placed, in the chamber there being provided both an air intake aperture to enable external air to enter the chamber and a delivery aperture for the exit of the atomized medicament stream, and a medicament atomization device, characterised in that the atomization device comprises an element of overall disc shape positioned within said chamber in such a manner as to dip into the liquid medicament contained in it, and means for rotating the disc element about its axis at a high r.p.m., the rotational speed of the disc element and its surface roughness being chosen such as to obtain the required atomization of the medicament.

In practice it has been found that to obtain the required medicament atomization it is convenient to fix the rotational speed of the disc element at a suitable value (for example 15000 r.p.m.) and then to choose a suitable roughness for the surface of the disc element (for example a roughness between 1 and 10 Ra in microns), to obtain the required atomization.

It has also been found that to achieve a better exit atomization of the medicament it is convenient to provide, within the atomization chamber, jet-breaking means for intercepting and disintegrating the larger medicament particles which are propelled by the action of the disc element which dips into the medicament.

The invention will be more apparent from the following description of one embodiment thereof. In this description reference is made to the accompanying drawing, in which:
Figure 1 is a very schematic section through the aerosol therapy apparatus of the present invention, taken on a vertical plane perpendicular to the axis of rotation of the disc element and centered on this latter; and
Figure 2 is a cross-section therethrough on the line 2-2 of Figure 1.

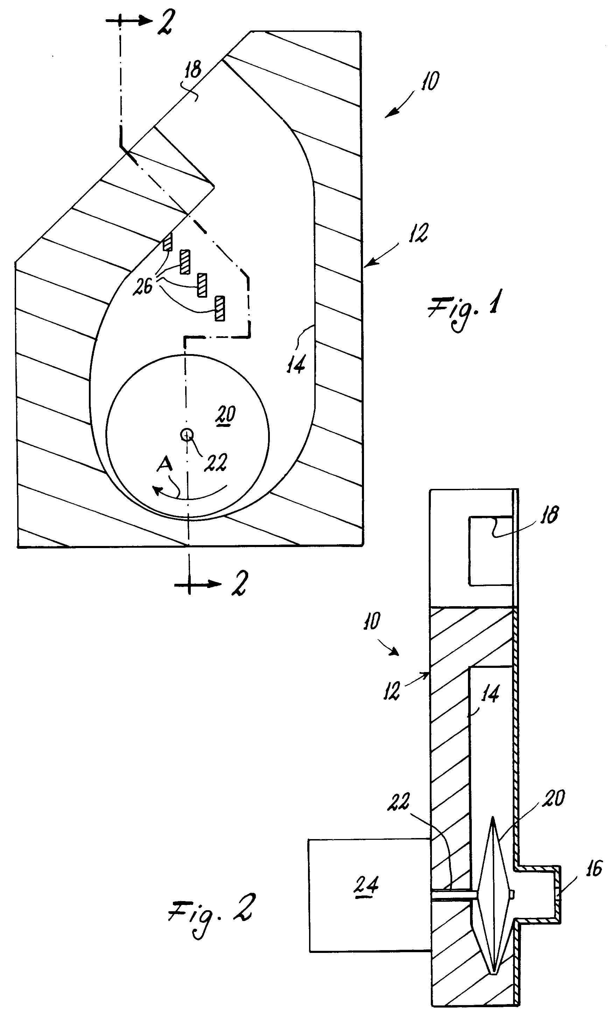

As can be seen from the figures, the aerosol therapy apparatus 10 comprises a hollow body 12 containing the cavity 14. This latter communicates with the outside both via an air intake aperture 16 (Figure 2) and via a delivery aperture 18 which enables the atomized medicament stream formed in the chamber 14 to emerge. A conventional mask or nasal connector or mouthpiece (not shown), of the type provided with known aerosol therapy apparatus, is connected to the delivery aperture 18.

In the lower part of the atomization chamber 14 there is provided a disc-shaped element 20 lying in a vertical plane and fixed onto a coaxial shaft 22. As can be seen from Figure 2, the base of the chamber 14 is of V cross-section, whereas the shape of the lower edge of the chamber 14 when viewed laterally (Figure 1) is curved and nearly tangential to the peripheral edge of the disc 20. This latter can rotate clockwise with respect to Figure 1, as indicated by the arrow A. The shaft 22 can be rotated by an electric motor 24 represented very schematically in Figure 2.

As can be seen from Figure 1, the atomization chamber 14 is traversed by a series of parallel blades 26 acting as a "jet-breaking" means the purpose of which, as stated, is to intercept and disintegrate the larger medicament particles propelled upwards by the disc when rotating and dipping into the liquid medicament (not shown) which occupies the lower part of the chamber 14. By virtue of the blades 26, better atomization of the medicament is obtained.

When the electric motor 24 is operated after loading the dose of liquid medicament into the chamber 14 through the aperture 18, the disc 20 rotates (for example at 15000 r.p.m.) to result in atomization of the medicament. The rotation of the disc 20 creates within the chamber 14 a vacuum which, combined with the inhalation exerted by the patient through the delivery aperture 18, results in the passage from this latter of an air stream which conveys the atomized medicament.

Both the disc 20 and the inner walls of the atomization chamber 14 are of a suitable material compatible with the use for which it is intended. As already stated, the disc 20 has a surface of suitable roughness, for example between 1 and 10 Ra (arithmetical mean deviation of the profile, in microns). The most suitable roughness can be chosen following experimental tests once the rotational speed of the disc has been fixed. As will be apparent, the apparatus of the invention delivers per unit of time a quantity of atomized medicament which decreases as the level of medicament contained in the chamber 14 decreases, until it becomes zero. This however does not create a problem in its use. In order to reduce this delivery variation as much as possible, the lower part of the atomization chamber is shaped as shown in the figures. This V shape in practice enables the entire dose of medicament loaded into the chamber 14 to be delivered at a virtually constant rate.

As will be apparent, the aerosol therapy apparatus according to the present invention has an extremely simple structure and is much less bulky than the apparatus currently used for this therapy. Its structural simplicity enables it to be produced on a large scale at substantially lesser cost than that of known apparatus. Because of its constructional and operational simplicity, this apparatus is also highly reliable, has a long life and does not require special maintenance beyond normal cleaning. Finally, it is able to achieve a substantially higher atomization rate than known aerosol therapy apparatus, because of which the time required for the patient to inhale the medicament dose is significantly reduced.

## Claims

1. An aerosol therapy apparatus (10) comprising an atomization chamber (14) in which the dose of liquid medicament to be atomized is placed, in the chamber (14) there being provided both an air intake aperture (16) to enable external air to enter the chamber (14) and a delivery aperture (18) for the exit of the atomized medicament stream, and a medicament atomization device, characterised in that the atomization device comprises an element (20) of overall disc shape positioned within the chamber (14) in such a manner as to dip into the liquid medicament contained in it, and means (24) for rotating the disc element (20) about its axis (22) at a high r.p.m., the rotational speed of the disc element (20) and its surface roughness being chosen such as to obtain the required atomization of the medicament.

2. An apparatus (10) as claimed in claim 1, wherein at least a part of the surface of the disc element (20) is rough.

3. An apparatus (10) as claimed in claim 2, wherein the rotational speed of the disc element (20) is about 15,000 r.p.m. and its surface roughness is between 1 and 10 Ra.

4. An apparatus (10) as claimed in any one of the preceding claims, wherein jet-breaking means (26) are provided in the atomization chamber (14) to disintegrate the larger liquid medicament particles propelled upwards by the disc element (20) when rotating and dipping into the liquid medicament.

5. An apparatus (10) as claimed in claim 4, wherein the jet-breaking means (26) consist of a series of parallel blades (26).

6. An apparatus (10) as claimed in any one of the preceding claims, wherein the disc element (20) is in the form of two coaxial identical cone frusta joined together at their base.

7. An apparatus (10) as claimed in claim 6, wherein the lower part of the atomization chamber (14) has a cross-section, taken on a vertical plane passing through the axis (22) of the disc element (20), which is of V profile centered on the centre plane through the disc element (20), the angle formed by the two conical lateral surfaces of the disc element (20) being slightly less than the angle enclosed by the V.
